# EUROPÄISCHE PATENTSCHRIFT

(11) **EP 2 280 947 B1**
(45) Veröffentlichungstag und Bekanntmachung des Hinweises auf die Patenterteilung: **26.11.2014**
(21) Anmeldenummer: 09738166.9
(22) Anmeldetag: 29.04.2009
(51) Int. Cl.: C07D 235/18

(54) **HERSTELLUNG VON 1,7´-DIMETHYL-2´-PROPYL-2,5´-BI-1H-BENZIMIDAZOL**
PRODUCTION OF 1,7´-DIMETHYL-2´-PROPYL-2,5´-BI-1H-BENZIMIDAZOLE
PROCÉDÉ DE FABRICATION DE 1,7'-DIMÉTHYL-2'-PROPYL-2,5'-BIS-1H-BENZIMIDAZOLE

(30) Priorität: 02.05.2008 EP 08155560
(43) Veröffentlichungstag der Anmeldung: 09.02.2011
(73) Patentinhaber: Boehringer Ingelheim International GmbH, 55216 Ingelheim am Rhein (DE)
(72) Erfinder: EHLENZ, Richard, 55216 Ingelheim Am Rhein (DE); MEYER, Oliver, 55216 Ingelheim am Rhein (DE); SCHMIDT, Hartmut, 55216 Ingelheim am Rhein (DE)
(74) Vertreter: Simon, Elke Anna Maria
(86) Internationale Anmeldenummer: PCT/EP2009/055162
(87) Internationale Veröffentlichungsnummer: WO 2009/133122

(56) Entgegenhaltungen:
- WO-A-03/059890

## Beschreibung

Die Erfindung betrifft an Position 2 substituierte Benzimidazole.

1,7'-Dimethyl-2'-propyl-2,5'-bi-1*H*-benzimidazol wird als Zwischenprodukt in der großtechnischen Synthese von Telmisartan verwendet. Dessen Verwendung als Pharmawirkstoff erfordert, dass das zur Synthese verwendete 1,7'-Dimethyl-2'-propyl-2,5'-bi-1*H*-benzimidazol mit einem sehr hohen Reinheitsgrad verwendet wird.

WO 03/059890 beschreibt die Herstellung von 1,7'-Dimethyl-2'-propyl-2,5'-bi-1*H-*benzimidazol durch Umsetzung von 2-Propyl-4-methyl-1*H*-benzimidazol-6-carbonsäure mit N-Methyl-o-phenylen-diamin in Gegenwart von Methansulfonsäure und Phosphorpentoxid. Gemäss dem dort beschriebenen Verfahren werden die Edukte N-Methyl-o-phenylen-diamin der Formel (II) oder dessen Salze 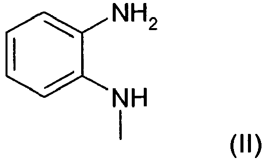 und 2-Propyl-4-methyl-1H-benzimidazol-6-carbonsäure der Formel (III) oder deren Salzen 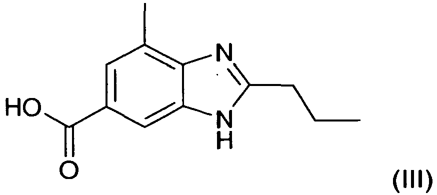 in Gegenwart von Phosphorpentoxid in Methansulfonsäure zu 1,7'-Dimethyl-2'-propyl-2,5'-bi-1 H-benzimidazol umgesetzt, wobei die einzelnen

Reaktionskomponenten in der folgenden Reihenfolge und bei folgenden Temperaturbereichen eingesetzt werden:
Phosphorpentoxid wird in Methansulfonsäure vorgelegt. Dann wird 2-Propyl-4-methyl-1 H-benzimidazol-6-carbonsäure gefolgt von N-Methyl-o-phenylen-diamin in Methansulfonsäure bei einer maximalen Temperatur von 150°C zudosiert.
Gegenstand der vorliegenden Erfindung ist ein dazu alternatives Verfahren zur Herstellung von 1,7'-Dimethyl-2'-propyl-2,5'-bi-1H-benzimidazol der Formel (I)

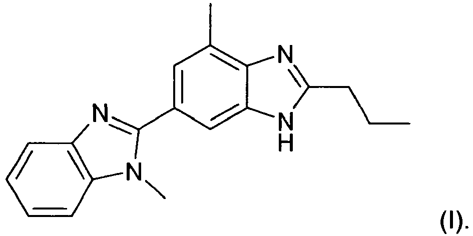

In diesem Verfahren werden N-Methyl-o-phenylen-diamin der Formel (II) oder dessen Salze 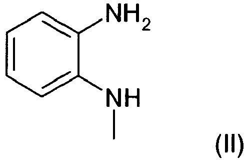 mit 2-Propyl-4-methyl-1H-benzimidazol-6-carbonsäure der Formel (III) oder deren Salzen 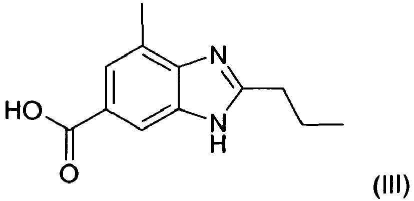 umgesetzt wobei
(a) die Verbindung der Formel (III) oder eines seiner Salze in einem polaren Lösungsmittel gelöst wird,
(b) dieser Lösung die Verbindung der Formel (II) oder eines seiner Salze zugegeben wird,
(c) anschliessend dieser Mischung der beiden Edukte in einem polaren Lösungsmittel Phosphorpentoxid zugegeben wird, und
(d) die Reaktion in einem Temperaturbereich von bis zu 160°C bevorzugt bei 110-160°C durchgeführt wird.

Außer der Verbindung der Formel (II) können in diesem Verfahren auch dessen Salze verwendet werden. Bevorzugte Salze sind das Phosphat-, Perchlorat, Chlor- oder Bromsalz. Besonders bevorzugt ist das Phospharsalz . Letzteres lässt sich beschreiben durch die Formel

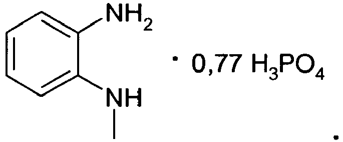

Analog können anstelle der 2-Propyl-4-methyl-1H-benzimidazol-6-carbonsäure der Formel (III) auch deren Salze verwendet werden. Bevorzugte Salze sind die Salze mit Natrium oder Kalium. Besonders bevorzugt ist die freie Carbonsäure.

So wird zum Beispiel
(a) Lösungsmittel vorgelegt,
(b) 2-Propyl-4-methyl-1 H-benzimidazol-6-carbonsäure der Formel (III) zugegeben, bevorzugt über eine Zeitspanne von wenigstens 15 Minuten,
(c) N-Methyl-o-phenylen-diamin der Formel (II) zugegeben, bevorzugt über eine Zeitspanne von wenigstens 15 Minuten,
(d) Phosphorpentoxid zugegeben, bevorzugt über eine Zeitspanne von wenigstens 15 Minuten,
(e) der Ansatz wenigstens 1 Stunde bei maximal 160°C gehalten, bevorzugt bei 130°C bis 160°C,
(f) danach der Ansatz bevorzugt unter WasserKühlung verdünnt und mit Base auf ein pH-Wert von 0-3 eingestellt, mit Kohle behandelt und schliesslich
(g) das Produkt 1,7'-Dimethyl-2'-propyl-2,5'-bi-1H-benzimidazol mit einem Lösungsmittel wie Isopropanol extrahiert, und nach Zugabe eines Antisolvens wie Wasser isoliert.

Als Lösungsmittel in Schritt (a) eignen sich für dieses Verfahren polare Lösungsmittel wie *N,N*-Dimethylformamid, *N,N*-Dimethylacetamid, Dimethylsulfoxid, Sulfonsäurederivate wie Methansulfonsäure, Methanol, Ethanol und 2-Propanol, bevorzugt Methanol, Ethanol und Methansulfonsäure.

Als Lösungsmittel zur Extraktion in Schritt (g) eignen sich höherwertige Alkohole wie Isopropanol sowie mit Wasser als Antisolvens nicht mischbare Lösungsmittel.

Die Schritte (a) und (b) werden bevorzugt in einem Temperaturbereich von 50°C bis 160°C, besonders bevorzugt bei 75°C bis 85°C durchgeführt.

Schritt (c) wird bevorzugt in einem Temperaturbereich von 50°C bis 160°C, besonders bevorzugt bei 85°C bis 95°C durchgeführt.

Schritt (d) wird bevorzugt in einem Temperaturbereich von maximal 160°C, besonders bevorzugt bei 110°C bis 130°C durchgeführt.

Die erhaltene Verbindung 1,7'-Dimethyl-2'-propyl-2,5'-bi-1 H-benzimidazol wird aufgereinigt, indem das Produkt mit Kohle behandelt wird, in 2-Propanol extrahiert und aus Isopropanol / Wasser isoliert wird. Der Reinheitsgrad von über 98% wird durch HPLC Messung ermittelt und beträgt zwischen 99% und 100% vorzugsweise 99,5%. Im Vergleich zum herkömmlichen Verfahren wird damit eine Verbesserung im Bezug auf die Summe der Verunreinigungen um den Faktor zwei erzielt.

Die Herstellung von 1,7'-Dimethyl-2'-propyl-2,5'-bi-1H-benzimidazol nach dem beschriebenen Verfahren ist effizienter, da Ausbeuten von mehr als 80%, vorzugsweise mehr als 85%, isoliert werden können und die Produktreinheit die des herkömmlichen Verfahrens deutlich übertrifft.

### Beispiele:

### Beipiel 1: Referenzbeispiel

Methansulfonsäure wird auf eine Temperatur von 115°C bis 130° C erwärmt. Phosphorpentoxid wird bei einer maximalen Temperatur von 145°C eingetragen. 2-Propyl-4-methyl-1H-benzimidazol-6-carbonsäure wird bei etwa 130-135°C eingetragen. Schließlich wird N-Methyl-o-phenylen-diamin bei etwa 135°C eingetragen. Der Ansatz wird dann 3 Stunden bei maximal 145°C nachgerührt. Es wird auf <100°C gekühlt und Wasser zum Reaktionsgemisch zudosiert. 50%ige Natronlauge wird bei <100°C bis zu einem pH-Wert kleiner 3 zugegeben. Schließlich werden bei < 100°C Behandlungen mit Kohle durchgeführt.

Bei einer Temperatur von < 80°C wird Isopropanol eingetragen und mit Natronlauge auf einen pH-Wert zwischen 4,5 und 7 eingestellt. Die wässrige Phase wird abgetrennt.

Zur Fällung von Dimethyl-2'-propyl-2,5'-bi-1H-benzimidazol wird Wasser zudosiert, der Apparateinhalt aus technischen Gründen auf mindestens 40°C gekühlt und das Produkt isoliert.
Ausbeute: 74-85 % der Theorie
HPLC-Reinheit: >99,5 %.

### Beipiel 2: Variante 2

Methansulfonsäure wird auf etwa 80°C erwärmt. Bei einer Temperatur von 75°C bis 85°C wird 2-Propyl-4-methyl-1 H-benzimidazol-6-carbonsäure eingetragen. Anschließend wird bei 85°C bis 95°C N-Methyl-o-phenylen-diamin eingetragen. Der Ansatz wird auf 110°C bis 130°C erwärmt und Phosphorpentoxid bis maximal 160°C Innentemperatur zudosiert. Anschließend wird der Ansatz 3 Stunden bei maximal 145°C nachgerührt. Es wird auf <100°C gekühlt und Wasser zum Reaktionsgemisch zudosiert. 50%ige Natronlauge wird bei <100°C bis zu einem pH-Wert kleiner 3 zugegeben.

Schließlich werden bei <100°C Behandlungen mit Kohle durchgeführt.

Bei einer Temperatur von <80°C wird Isopropanol eingetragen und mit Natronlauge auf einen pH-Wert zwischen 4,5 und 7 eingestellt. Die wässrige Phase wird abgetrennt.

Zur Fällung von Dimethyl-2'-propyl-2,5'-bi-1*H*-benzimidazol wird Wasser zudosiert, der Apparateinhalt aus technischen Gründen auf mindestens 40°C gekühlt und das Produkt isoliert.
Ausbeute: 78-90 % der Theorie
HPLC Reinheit: > 99,5 %.

## Patentansprüche

1. Verfahren zur Herstellung von 1,7'-Dimethyl-2'-propyl-2,5'-bi-1H-benzimidazol der Formel (I) 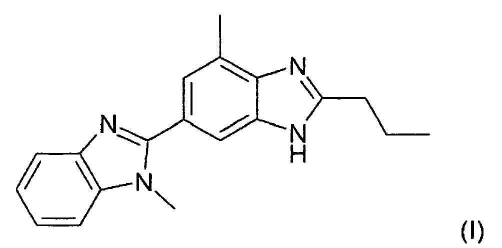 durch Umsetzung von N-Methyl-o-phenylen-diamin der Formel (II) oder dessen Salzen 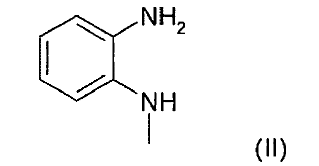 mit 2-Propyl-4-methyl-1 H-benzimidazol-6-carbonsäure der Formel (III) oder deren Salzen 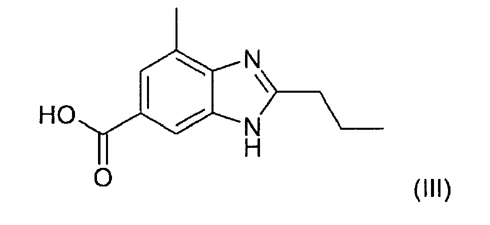 **dadurch gekennzeichnet dass**
(a) die Verbindung der Formel (III) oder eines seiner Salze in einem polaren Lösungsmittel gelöst wird,
(b) dieser Lösung die Verbindung der Formel (II) oder eines seiner Salze zugegeben wird,
(c) anschliessend dieser Mischung der beiden Edukte in einem polaren Lösungsmittel Phosphorpentoxid zugegeben wird, und
(d) die Reaktion in einem Temperaturbereich von bis zu 160°C durchgeführt wird.

2. Verfahren Anspruch 1 **dadurch gekennzeichnet dass** die Reaktion gemäss Schritt (d) bei 110-160°C durchgeführt wird.

3. Verfahren nach Anspruch 1 **dadurch gekennzeichnet dass** 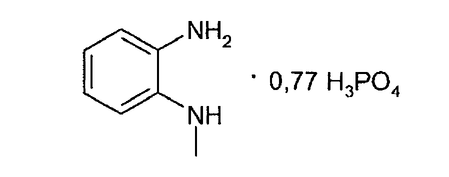 als Salz der Verbindung der Formel (II) verwendet wird.

4. Verfahren nach Anspruch 1 **dadurch gekennzeichnet, dass**
(a) Lösungsmittel vorgelegt wird,
(b) 2-Propyl-4-methyl-1 H-benzimidazol-6-carbonsäure der Formel (III) zugegeben wird,
(c) N-Methyl-o-phenylen-diamin der Formel (II) zugegeben wird,
(d) Phosphorpentoxid zugegeben wird,
(e) der Ansatz wenigstens 1 Stunde bei maximal 160°C gehalten wird,
(f) danach der Ansatz verdünnt und mit Base auf ein pH-Wert von 0-3 eingestellt wird, und schließlich
(g) das Produkt 1,7'-Dimethyl-2'-propyl-2,5'-bi-1 H-benzimidazol mit Kohle behandelt wird, mit einem Lösungsmittel wie Isopropanol extrahiert, und aus Isopropanol / Wasser isoliert wird.

5. Verfahren nach Anspruch 1 **dadurch gekennzeichnet dass** ein polares Lösungsmittel wie *N,N*-Dimethylformamid, *N,N*-Dimethylacetamid, Sulfonsäurederivate wie Methansulfonsäure, Dimethylsulfoxid, Methanol, Ethanol oder 2-Propanol verwendet wird.

6. Verfahren nach Anspruch 4 **dadurch gekennzeichnet dass** Methansulfonsäure als polares Lösungsmittel verwendet wird.

7. Verfahren nach Anspruch 6 **dadurch gekennzeichnet dass**
Schritt (a) bei einer Temperatur von 50°C bis 160°C,
Schritt (b) bei einer Temperatur von 50°C bis 160°C,
Schritt (c) bei einer Temperatur von 50°C bis 160°C, und
Schritt (d) bei einer Temperatur von maximal 160°C durchgeführt werden.

8. Verfahren nach Anspruch 7 **dadurch gekennzeichnet dass**
Schritt (a) bei 75-85°C,
Schritt (b) bei 75-85°C,
Schritt (c) bei 85-95°C und
Schritt (d) bei 110-130°C durchgeführt werden.

9. Verfahren nach Anspruch 1 **dadurch gekennzeichnet dass** eine Ausbeute von mehr als 80% erreicht wird.

10. Verfahren nach Anspruch 1 **dadurch gekennzeichnet dass** der Reinheitsgrad von 1,7'-Dimethyl-2'-propyl-2,5'-bi-1 H-benzimidazol 98% übersteigt.

## Claims

1. Process for preparing 1,7'-dimethyl-2'-propyl-2,5'-bi-1 H-benzimidazole of formula (I) 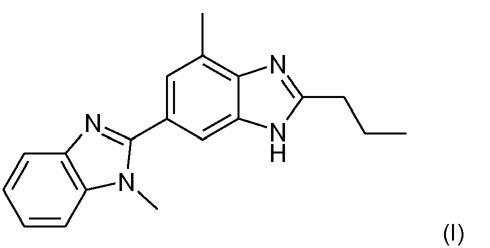 by reacting N-methyl-o-phenylene-diamine of formula (II) or the salts thereof 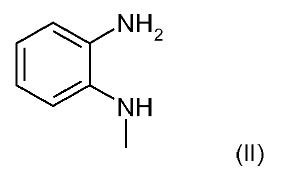 with 2-propyl-4-methyl-1 H-benzimidazole-6-carboxylic acid of formula (III) or the salts thereof 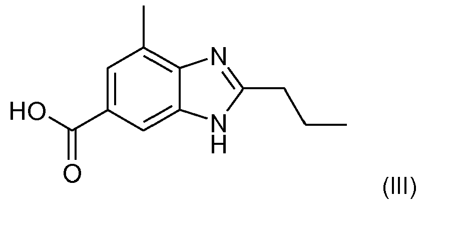 **characterised in that**
(a) the compound of formula (III) or one of the salts thereof is dissolved in a polar solvent,
(b) the compound of formula (II) or one of the salts thereof is added to this solution,
(c) then phosphorus pentoxide is added to this mixture of the two starting compounds in a polar solvent, and
(d) the reaction is carried out in a temperature range of up to 160°C.

2. Process according to claim 1, **characterised in that** the reaction according to step (d) is carried out at 110-160°C.

3. Process according to claim 1, **characterised in that** 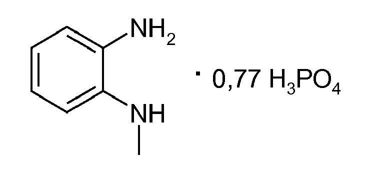 is used as the salt of the compound of formula (II).

4. Process according to claim 1, **characterised in that**
(a) solvent is taken,
(b) 2-propyl-4-methyl-1H-benzimidazole-6-carboxylic acid of formula (III) is added,
(c) N-methyl-o-phenylene-diamine of formula (II) is added,
(d) phosphorus pentoxide is added,
(e) the mixture is kept at a maximum temperature of 160°C for at least 1 hour,
(f) then the mixture is diluted and adjusted to a pH of 0-3 with base, and finally
(g) the product 1,7'-dimethyl-2'-propyl-2,5'-bi-1 H-benzimidazole is treated with charcoal, extracted with a solvent such as isopropanol, and isolated from isopropanol / water.

5. Process according to claim 1, **characterised in that** a polar solvent such as N,N-dimethylformamide, N,N-dimethylacetamide, sulphonic acid derivatives such as methanesulphonic acid, dimethylsulphoxide, methanol, ethanol or 2-propanol is used.

6. Process according to claim 4, **characterised in that** methanesulphonic acid is used as polar solvent.

7. Process according to claim 6, **characterised in that**
step (a) is carried out at a temperature of 50°C to 160°C,
step (b) is carried out at a temperature of 50°C to 160°C,
step (c) is carried out at a temperature of 50°C to 160°C, and
step (d) is carried out at a temperature of not more than 160°C.

8. Process according to claim 7, **characterised in that**
step (a) is carried out at 75-85°C,
step (b) is carried out at 75-85°C,
step (c) is carried out at 85-95°C, and
step (d) is carried out at 110-130°C.

9. Process according to claim 1, **characterised in that** a yield of more than 80% is achieved.

10. Process according to claim 1, **characterised in that** the degree of purity of 1,7'-dimethyl-2'-propyl-2,5'-bi-1H-benzimidazole exceeds 98%.

## Revendications

1. Procédé de production de 1,7'-diméthyl-2'-propyl-2,5'-bi-1H-benzimidazole de formule (I) 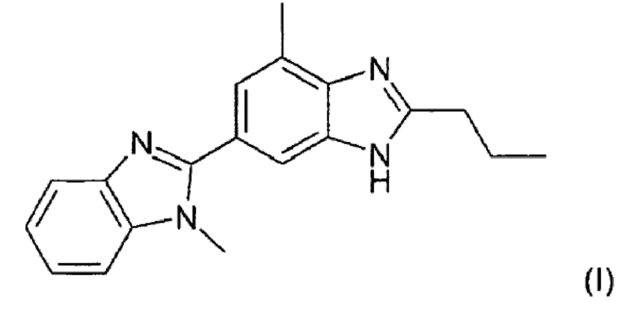 en faisant réagir du N-méthyl-o-phénylène-diamine de formule (II) ou ses sels 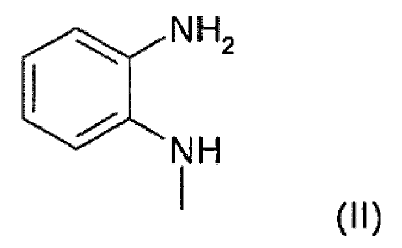 avec de l'acide 2-propyl-4-méthyl-1H-benzimidazole-6-carboxylique de formule (III) ou avec ses sels 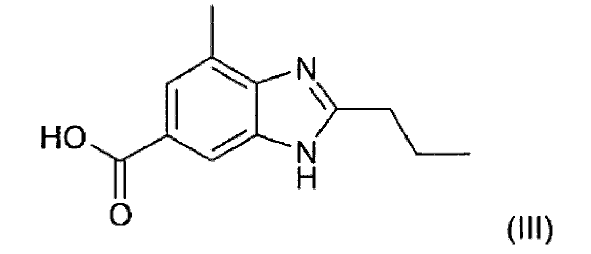 **caractérisé en ce que**
(a) le composé de formule (III) ou un de ses sels est dissous dans un solvant polaire,
(b) le composé de formule (II) ou un de ses sels est ajouté à ladite solution,
(c) du pentoxyde de phosphore est ajouté immédiatement audit mélange des deux produits de départ dans un solvant polaire, et
(d) la réaction est effectuée dans une plage de températures allant jusqu'à 160°C.

2. Procédé selon la revendication 1, **caractérisé en ce que** la réaction est effectuée selon l'étape (d) à une température comprise entre 110 et 160°C.

3. Procédé selon la revendication 1, **caractérisé en ce qu'**on utilise 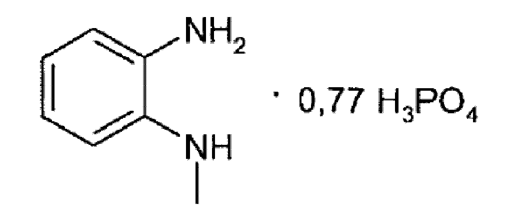 comme sel du composé de formule (II).

4. Procédé selon la revendication 1, **caractérisé en ce que**
(a) le solvant est déposé,
(b) de l'acide 2-propyl-4-méthyl-1H-benzimidazole-6-carboxylique de formule (III) est ajouté,
(c) du N-méthyl-o-phénylène-diamine de formule (II) est ajouté,
(d) du pentoxyde de phosphore est ajouté,
(e) la préparation est maintenue durant au moins 1 heure à une température de 160°C au maximum,
(f) puis la préparation est diluée et sa valeur de pH est ajustée avec une base à une valeur allant de 0 à 3, et pour finir
(g) le produit 1,7'-diméthyl-2'-propyl-2,5'-bi-1H-benzimidazole est traité avec du charbon, est extrait avec un solvant tel que l'isopropanol et est isolé à partir d'isopropanol/eau.

5. Procédé selon la revendication 1, **caractérisé en ce qu'**on utilise un solvant polaire tel que le N,N-diméthylformamide, le N,N-diméthylacétamide, des dérivés d'acide sulfonique tels que l'acide méthane sulfonique, le diméthylsulfoxyde, le méthanol, l'éthanol ou le 2-propanol.

6. Procédé selon la revendication 4, **caractérisé en ce qu'**on utilise comme solvant polaire de l'acide méthane sulfonique.

7. Procédé selon la revendication 6, **caractérisé en ce que** l'étape (a) est effectuée à une température allant de 50°C à 160°C,
l'étape (b) est effectuée à une température allant de 50°C à 160°C,
l'étape (c) est effectuée à une température allant de 50°C à 160°C, et
l'étape (d) est effectuée à une température de 160°C au maximum.

8. Procédé selon la revendication 7, **caractérisé en ce que** l'étape a) est effectuée à une température allant de 75 à 85°C,
l'étape (b) est effectuée à une température allant de 75 à 85°C,
l'étape (c) est effectuée à une température allant de 85 à 95°C et
l'étape (d) est effectuée à une température allant de 110 à 130°C.

9. Procédé selon la revendication 1, **caractérisé en ce qu'**on atteint un rendement supérieur à 80 %.

10. Procédé selon la revendication 1, **caractérisé en ce que** le degré de pureté du 1,7'-diméthyl-2'-propyl-2,5'-bi-1H-benzimidazole dépasse 98 %.
